# EUROPÄISCHE PATENTANMELDUNG

(11) **EP 4 552 674 A1**
(43) Veröffentlichungstag der Anmeldung: **14.05.2025**
(21) Anmeldenummer: 24189804.8
(22) Anmeldetag: 19.07.2024
(51) Int. Cl.: A61M 5/315, A61M 5/20, A61M 5/32, A61M 5/24

(54) **AUTOINJEKTOR MIT EINER VERRIEGELUNGSHÜLSE**

(30) Priorität: 07.11.2023 EP 23208238
(71) Anmelder: Ypsomed AG, 3401 Burgdorf (CH)
(72) Erfinder: Tschirren, Markus, 3400 Burgdorf (CH); Mellenberger, Andres, 3400 Burgdorf (CH)
(74) Vertreter: Eugster, Monika Katharina

(57) **Zusammenfassung**

Die Erfindung betrifft einen Autoinjektor zur Ausschüttung eines flüssigen Produkts mit einem Gehäuse (2) und einer Sperrhülse (7), wobei eine Verriegelungseinrichtung an der Sperrhülse (7) und an einem gehäusefesten Mechanikhalter (5; 5'; 5") vorgesehen ist. Die Verriegelungseinrichtung dient dazu, dass eine Nadelschutzhülse (3) in einer Nadelschutzposition verriegelt wird, wobei in der Nadelschutzposition die Nadelschutzhülse (3) relativ zu dem Mechanikhalter (5; 5'; 5") gegen ein Zurückschieben in die proximale Richtung so verriegelt, dass die eine Nadelspitze einer Nadel (11a) nicht aus dem distalen Ende der Nadelschutzhülse (3) hervortreten kann.

## Beschreibung

Die Erfindung betrifft einen Autoinjektor, der oftmals auch als Autoinjektionsvorrichtung bezeichnet wird, mit dem ein in einem Produktbehälter enthaltenes Produkt nach dem Auslösen automatisch ausschüttbar ist. Bei dem flüssigen Produkt handelt es sich insbesondere um ein Medikament. Im Besonderen betrifft die Erfindung einen Autoinjektor mit einer Sperrhülse, wobei der Autoinjektor nach einer Auslösung nicht erneut ausgelöst werden kann.

Der Begriff "Medikament" umfasst hier jede fliessfähige medizinische Formulierung, welche geeignet ist zur kontrollierten Verabreichung durch ein Mittel wie eine Kanüle oder Nadel, insbesondere eine Hohlnadel hindurch, beispielsweise umfassend eine Flüssigkeit, eine Lösung, ein Gel, oder eine feine Suspension, welche einen oder mehrere medizinische Wirkstoffe enthält. Medikament kann eine Zusammensetzung mit einem einzigen Wirkstoff oder eine vorgemischte oder co-formulierte Zusammensetzung mit mehreren Wirkstoffen aus einem einzelnen Behälter sein. Medikament umfasst Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzymen (z.B. Hydrolasen, Lyasen) und weitere Substanzen sowohl in fester (suspendierter) oder flüssiger Form aber auch Polysaccaride (z.B. Glycosaminoglycan), Vaccine, DNS oder RNS oder Oglionukleotide, Antikörper oder Teile von Antikörpern sowie geeignete Basis-, Hilfs- und Trägerstoffe.

Autoinjektoren sind aus dem Stand der Technik, wie aus der EP2742962B2, bekannt, um einfach und sicher mit einer vorgespannten Feder eine Injektion abzugeben, wobei nach Abgabe der Injektion keine weitere Injektion abgegeben werden kann.

Es ist eine Aufgabe der Erfindung, einen alternativen Autoinjektor anzugeben, welcher nach Abgabe einer Injektion keine weitere Abgabe erlaubt.

Die Aufgabe wird mit dem Autoinjektor nach Anspruch 1 gelöst. Vorteilhafte Weiterentwicklungen ergeben sich aus den abhängigen Ansprüchen, der Beschreibung und den Figuren.

Der erfindungsgemäße Autoinjektor weist ein Gehäuse und einen in dem Gehäuse angeordneten Produktbehälter auf. Bei dem Produktbehälter kann es sich insbesondere um eine Spritze handeln, welche einen Spritzenkörper aufweist, an dessen distalem Ende eine Nadel fest angeordnet ist. Der vorzugsweise zylindrische Spritzenkörper umgibt einen Kolben, der in Bezug auf den Spritzenkörper verschiebbar ist und für die Produktausschüttung zu dem distalen Ende hin verschoben wird, wodurch das zwischen dem Kolben und der Nadel angeordnete flüssige Produkt, insbesondere Medikament, durch die Nadel aus dem Produktbehälter ausgeschüttet wird. Der Spritzenkörper kann an seinem proximalen, d. h. hinteren oder der Nadel entgegengesetzten Ende einen Flansch, der auch als Fingerflansch bezeichnet werden kann, aufweisen. Eine derartig ausgebildete Spritze ist als Standardspritze erhältlich, so dass für den Autoinjektor nicht zwingend eine speziell angepasste Spritze entwickelt zu werden braucht. Der Kolben liegt dichtend an dem Innendurchmesser des Spritzenkörpers an.

Das Gehäuse ist vorzugsweise länglich und bildet die Längsachse des Autoinjektors. Das Gehäuse ist vorzugsweise hülsenförmig und/oder zylindrisch, insbesondere kreiszylindrisch oder oval. Der Produktbehälter ist in dem Gehäuse angeordnet. Beispielsweise kann der Behälter verschiebbar in dem Gehäuse angeordnet sein, d. h. für ein automatisches Einstechen relativ zu dem Gehäuse in distale Richtung verschiebbar sein, so dass die Nadelspitze aus einer Öffnung am distalen Ende des Autoinjektors hervortritt und automatisch in den Patienten eingestochen werden kann. Optional kann bei einer solchen Vorrichtung die Nadelspitze nach erfolgter Produktausschüttung in das distale Ende der Vorrichtung hinein bewegt werden, insbesondere der Produktbehälter relativ zu dem Gehäuse in die proximale Richtung bewegt werden.

In bevorzugten Ausführungen ist der Produktbehälter entlang der Längsachse unverschiebbar in dem Gehäuse aufgenommen, insbesondere mittels eines Produktbehälterhalters oder Spritzenhalters, der den Produktbehälter oder die Spritze axialfest hält und der axialfest mit dem Gehäuse verbunden, insbesondere verrastet ist. Bevorzugt steht die Nadelspitze über das distale Ende des Gehäuses in distale Richtung über. Hierdurch kann die Nadel mittels einer Bewegung des Gehäuses zum Patienten hin in die Einstichstelle eingestochen werden. Vorzugsweise ist eine Nadelschutzhülse vorgesehen, welche das distale Ende des Autoinjektors bildet und eine Öffnung für die Nadel aufweist, wobei die Nadel durch die Öffnung hindurchtreten kann. Die Nadelschutzhülse ist vorzugsweise relativ zu dem Gehäuse drehfest und axial bewegbar angeordnet. Die Nadelschutzhülse kann in ihrer Ausgangsposition in Bezug auf die Nadelspitze so angeordnet sein, dass die Nadelschutzhülse distal über die Nadelspitze steht oder dass die Nadelspitze distal über das distale Ende der Nadelschutzhülse steht. Die Nadelschutzhülse ist relativ zu dem Gehäuse aus ihrer Ausgangsposition in die proximale Richtung um einen Betätigungshub in eine betätigte Position verschiebbar, insbesondere in das Gehäuse verschiebbar, so dass die Nadel aus dem distalen Ende bzw. durch die Öffnung der Nadelschutzhülse hervortritt oder weiter hervortritt. Bevorzugt kann die Nadelschutzhülse um einen Nadelschutzhub aus der betätigten Position relativ zu dem Gehäuse in die distale Richtung in eine Nadelschutzposition verschoben werden, in der das distale Ende der Nadelschutzhülse distal über die Nadelspitze steht, um zu verhindern, dass nach erfolgter Verwendung der Vorrichtung bzw. nach erfolgter Produktausschüttung eine Verletzungsgefahr, die von einer freiliegenden Nadelspitze ausgehen würde, besteht. Die Nadelschutzhülse z. B. gegen die Kraft einer Nadelschutzfeder, in die proximale Richtung verschoben werden, wobei die Nadelschutzfeder die Nadelschutzhülse aus der betätigten Position in die distale Richtung, d. h. in die Nadelschutzposition verschieben kann. Der Betätigungshub und der Nadelschutzhub der Nadelschutzhülse können gleich lang und unterschiedlich lang sein.

Die Nadelschutzfeder wirkt auf die Nadelschutzhülse, wobei die Nadelschutzhülse für die Auslösung der Produktausschüttung aus ihrer Ausgangsposition relativ zu dem Gehäuse und entlang der Längsachse des Autoinjektors in die proximale Richtung, d. h. entgegen die Ausschüttrichtung verschiebbar ist, insbesondere um den Betätigungshub. Hierdurch wird die Nadelschutzfeder gespannt und vorzugsweise auch die Produktausschüttung, insbesondere die Bewegung des Vortriebsglieds in die Ausschüttrichtung, ausgelöst. Die Nadelschutzhülse wird vorzugsweise dadurch aus ihrer Ausgangsposition um den Betätigungshub in ihre betätigte Position bewegt, dass ihr distales Ende an die Einstichstelle des Patienten angedrückt wird, wobei das Gehäuse relativ zu der Nadelschutzhülse in Richtung Einstichstelle verschoben wird, so dass die Nadelschutzhülse den Betätigungshub relativ zu dem Gehäuse ausführt. Hierbei wird auch die aus dem distalen Ende der Nadelschutzhülse hervortretende Nadel in die Einstichstelle eingestochen. Nach der erfolgten Produktausschüttung, insbesondere nach z. B. einer kurzen Wartezeit, wie z. B. 3 bis 10 Sekunden, nachdem beispielsweise ein Ausschüttsignal oder alternativ kein Ausschüttsignal mehr erzeugt wurde bzw. das Ausschüttsignal verstummt ist, wird der Autoinjektor von der Einstichstelle abgenommen, wodurch die Nadelschutzhülse relativ zu dem Gehäuse aus ihrer betätigten Position um den Nadelschutzhub in die Nadelschutzposition verschoben wird, insbesondere mittels der in der Nadelschutzfeder gespeicherten Federenergie. Durch das Abnehmen des Autoinjektors von der Einstichstelle wird auch die Nadel aus der Einstichstelle gezogen.

Die Nadelschutzfeder kann z. B. eine als Druckfeder wirkende Wendelfeder sein. Die Nadelschutzfeder ist vorzugsweise aus Metall gebildet. Die Nadelschutzfeder kann sich mit ihrem proximalen Ende beispielsweise an dem Gehäuse oder an einem gehäusefesten Element, insbesondere an einer gehäusefesten Verschlusskappe abstützen. Die Verschlusskappe ist insbesondere an dem proximalen Ende des Gehäuses angeordnet. Die Verschlusskappe kann das proximale Ende des hülsenförmigen Gehäuses verschliessen. Die Verschlusskappe ist axialfest, insbesondere axial- und drehfest mit dem Gehäuse verbunden. Besonders bevorzugt stützt sich die Nadelschutzfeder mit ihrem distalen Ende an der Nadelschutzhülse oder an deinem Element, welches, insbesondere bei Verschiebung der Nadelschutzhülse relativ zu dem Gehäuse, mit der Nadelschutzhülse verschoben wird, ab. Beispielsweise kann das Element eine Sperrhülse, wie sie weiter unten beschrieben wird, sein. Das Element kann insbesondere kinematisch und/oder geometrisch zwischen der Nadelschutzhülse und der Nadelschutzfeder, insbesondere dem distalen Ende der Nadelschutzfeder angeordnet sein. Der Vorteil hierbei ist, dass die Nadelschutzhülse mittels der Nadelschutzfeder aus ihrer betätigten Position in die Nadelschutzposition verschiebbar ist. Die Nadelschutzfeder kann somit bevorzugt eine Doppelfunktion erfüllen, da sie zusätzlich die oben genannte Kraft auf die Sperrhülse ausübt.

Der Autoinjektor kann ein Verriegelungseinrichtung aufweisen, welche die Nadelschutzhülse in ihrer Nadelschutzposition insbesondere in Bezug auf das Gehäuse verriegelt und ein Zurückschieben der Nadelschutzhülse in die proximale Richtung oder in das Gehäuse blockiert. Die Verriegelungseinrichtung dient dazu, dass in der Nadelschutzposition die Nadelschutzhülse relativ zu dem Gehäuse gegen ein Zurückschieben in die proximale Richtung so verriegelt, dass die die Nadelspitze nicht aus dem distalen Ende der Nadelschutzhülse hervortreten kann. Die Verriegelungseinrichtung kann vorzugsweise ein federnd oder elastisch angeordnetes Verriegelungsglied und ein korrespondierenden Verriegelungsanschlag umfassen. Das Verriegelungseinrichtung verriegelt die Nadelschutzhülse zumindest so, dass die Nadel nicht aus dem distalen Ende der Nadelschutzhülse hervortreten kann. Die Nadelschutzhülse kann z. B. aus der Nadelschutzposition nur soweit in die proximale Richtung verschoben werden, dass die Nadelspitze nicht aus dem distalen Ende der Nadelschutzhülse hervortritt.

Der Autoinjektor umfasst ferner ein Vortriebsglied, das zumindest während der Produktausschüttung auf den Kolben wirkt, insbesondere an dem Kolben anliegt, und eine Ausschüttfeder, die auf das Vortriebsglied wirkt wie z. B. sich insbesondere mit ihrem distalen Ende an dem Vortriebsglied abstützt. Das Vortriebsglied kann z. B. hülsenförmig sein. Die Ausschüttfeder ist vorzugsweise innerhalb des hülsenförmigen Vortriebsglieds angeordnet. Alternativ kann die Ausschüttfeder ausserhalb des hülsenförmigen Vortriebsglieds angeordnet sein. Die Ausschüttfeder ist vorzugsweise eine als Druckfeder wirkende Wendelfeder, die vorzugsweise aus Metall gebildet ist. Die Ausschüttfeder ist so stark vorgespannt, insbesondere im Auslieferungszustand oder in der Ausgangsposition des Autoinjektors, dass sie bzw. die in ihr gespeicherte Energie ausreicht, um das Produkt aus dem Produktbehälter durch Verschieben des Vortriebsglieds um einen Ausschütthub im Wesentlichen vollständig auszuschütten. Durch die Verschiebung des Vortriebsglieds um den Ausschütthub wird auch der Kolben verschoben. Sofern im Auslieferungszustand oder in der Ausgangsposition zwischen dem Kolben und dem Vortriebsglied ein Abstand besteht, ist der Ausschütthub des Kolbens kleiner als der Ausschütthub des Vortriebsglieds, was bevorzugt ist, da der Kolben bis zur Verwendung unbelastet bleibt, wodurch eine ungewollte vorzeitige Produktausschüttung vermieden wird. Grundsätzlich ist es aber auch möglich, dass das Vortriebsglied im Auslieferungszustand oder in der Ausgangsposition und nicht erst bei der Produktausschüttung an dem Kolben anliegt. Sofern im Auslieferungszustand oder in der Ausgangsposition das Vortriebsglied an dem Kolben bereits anliegt, entspricht der Ausschütthub des Kolbens dem Ausschütthub des Vortriebsglieds. Der Ausschütthub des Vortriebsglieds und der Ausschütthub des Kolbens können gleich lang oder unterschiedliche lang sein. Das proximale Ende der Ausschüttfeder kann sich an dem Gehäuse oder an einem gehäusefesten Element, insbesondere an der Verschlusskappe des Autoinjektors abstützen. Das Gehäuse oder das gehäusefeste Element, insbesondere die Verschlusskappe kann eine Führung auf, sodass die Ausschüttfeder vorgespannt und/oder entspannt geführt oder gestützt ist, so dass die Ausschüttfeder nicht einknickt. Diese Führung ragt durch das Innere der Ausschüttfeder.

In bevorzugten Ausführungsformen kann der Autoinjektor einen Adapter aufweisen, welcher im Innern, insbesondere axial bewegbar im Innern des Vortriebglieds angeordnet ist. Der Adapter kann einerseits dazu dienen den Abstand bei der Montage des Autoinjektors zwischen dem Vortriebsglied und dem Kolben anzupassen, insbesondere zu verkleinern, da aufgrund der verschiedenen Füllvolumina der Spritzen der Kolben an unterschiedlicher Stelle innerhalb der Spritze angeordnet ist. Dazu kann der Adapter eine Zahnung aufweisen, welche mit einer an dem Vortriebglied angeordneten Gegenzahnung in einem Eingriff ist, wobei durch eine axiale Relativbewegung zwischen dem Adapter und dem Vortriebsglied der Abstand zwischen dem in der Spritze vorgesehenen Kolben und dem Adapter eingestellt werden kann. Alternativ kann anstelle einer Verwendung eines zusätzlichen Adapters, der Abstand zwischen dem Kolben in der Spritze und dem Vortriebsglieds durch die axiale Positionierung zwischen dem Vortriebsglied und dem Gehäuse oder einem gehäusefesten Element, insbesondere einem gehäusefesten Mechanikhalter realisiert werden. Das Vortriebsglied und das Gehäuse bzw. der Mechanikhalter können dazu eine Zahnung und eine korrespondierende Gegenzahnung aufweisen, um den Abstand zwischen dem Kolben der Spritze und dem Vortriebsglied einzustellen. Andererseits kann beim Start der Ausschüttung die Energie des axial bewegbaren Vortriebsglieds mit Hilfe des Adapters absorbiert oder reduziert werden. Der Adapter reduziert dabei die Gefahr von Glasbruch, welche auftritt, wenn beim Start der Ausschüttung das Vortriebsglied mit einer schlagartig auftretenden Aufprallenergie auf den Kolben auftrifft. Diese Energie wird durch die Reibung zwischen dem Adapter und dem Spritzenkörper zumindest teilweise absorbiert, wenn der Adapter auf den im Innern der Spritze angeordneten Kolben auftrifft. Dazu kann der Adapter ein Gewinde und das Vortriebsglied eine Gegengewinde aufweisen, wobei das Gewinde und das Gegengewinde in einem Eingriff sind und derart ausgebildet sind, dass der Adapter und das Vortriebsglied relativ zueinander bis zur Selbsthemmung axial bewegbar sind. Alternativ kann der Adapter und/oder das Vortriebsglied ein oder mehrere Quetschelemente bzw. Quetschgegenelemente aufweisen oder als Quetschelement bzw. Quetschgegenelement ausgebildet sein. Bevorzugt ist das Quetschelement derart ausgebildet, dass die Aufprallenergie des Vortriebsglieds auf den Kolben zumindest teilweise über die Quetschung des Quetschelements durch das Quetschgegenelement absorbiert wird. Alternativ kann der Adapter und das Vortriebsglied eine Zahnung und eine Gegenzahnung aufweisen, um die kinetische Energie des Vortriebsglieds zumindest teilweise zu absorbieren. Dadurch wird ein Dämpfungseffekt zwischen dem Kolben und dem Vortriebsglied erwirkt.

Im Auslieferungszustand oder in der Ausgangsposition kann zwischen dem Kolben und dem Adapter ein Abstand besteht, so dass der Ausschütthub des Kolbens kleiner als der Ausschütthub des Vortriebsglieds ist. Alternativ kann der Adapter im Auslieferungszustand oder in der Ausgangsposition und nicht erst bei der Produktausschüttung an dem Kolben anliegt, so dass der Ausschütthub des Kolbens und der Ausschütthub des Vortriebsglied gleich sind.

Der Mechanikhalter ist relativ zu dem Gehäuse axialfest, insbesondere axial- und drehfest angeordnet. Der Mechanikhalter ist vorzugsweise hülsenförmig ausgebildet. Besonders bevorzugt ist das distale Ende des Mechanikhalters als Haltefeder ausgebildet und dient dazu, den Produktbehälter, insbesondere die Spritze in dem Produktbehälterhalter, insbesondere dem Spritzenhalter in die distale Richtung vorzuspannen, um mögliche Längentoleranzen des Produktbehälters, insbesondere der Spritze auszugleichen.

Der Produktbehälter ist vorzugsweise als Spritze ausbildet und in dem Produktbehälterhalter, besonders bevorzugt in dem Spritzenhalter aufgenommen. Die Spritze weist den Spritzenkörper, den Kolben und die Nadel auf, wobei die Nadel z. B. unlösbar an einem Nadelhalteabschnitt der Spritze befestigt ist und der Kolben in dem zylindrischen Abschnitt des Spritzenkörpers verschiebbar angeordnet ist. Der Spritzenkörper umfasst ferner einen sich verjüngenden Abschnitt auf, der zwischen dem Nadelhalteabschnitt und dem zylindrischen Abschnitt angeordnet ist. Die Spritze weist ferner eine Nadelschutzkappe auf, welche z. B. ein sogenanntes soft needle shield oder vorzugsweise ein rigid needle shield sein kann. Ein soft needle shield ist vorzugsweise aus einem gummielastischen Kunststoff gebildet, wobei ein rigid needle shield aus einer Hülse aus Hartkunststoff gebildet ist, in der eine Hülse aus einem gummielastischen Kunststoff angeordnet ist. Die Hülse aus gummielastischem Kunststoff und die Hülse aus Hartkunststoff bilden zusammen das rigid needle shield. Die Nadelschutzkappe, welche die Nadel abdeckt, ist lösbar an dem Nadelhalteabschnitt befestigt und hält die Nadel vorzugsweise geschützt vor Schmutz und steril. Zwischen dem sich Spritzenkörpers, insbesondere dem sich verjüngenden Abschnitt des Spritzenkörpers und der Nadelschutzkappe ist ferner eine Lücke gebildet.

Der Spritzenhalter weist mindestens ein Eingriffsglied, insbesondere eine Schulter, an welcher sich der verjüngende Abschnitt der Spritze in die distale Richtung abstützt und welche in die Lücke zwischen der Nadelschutzkappe und dem sich verjüngenden Abschnitt eingreift, auf. Vorteilhaft wird durch das Anliegen des sich verjüngenden Abschnitts an dem Eingriffsglied verhindert, dass sich die Spritze relativ zu dem Spritzenhalter in die distale Richtung bewegen kann. Durch das Vorspannen des Mechanikhalters, insbesondere der Haltefeder des Mechanikhalters in die distale Richtung wird zudem der feste Sitz der Spritze an dem Eingriffsglied sichergestellt.

Das Gehäuse oder die Nadelschutzhülse des Autoinjektors kann vorzugsweise einen Halteabschnitt aufweisen, der an dem Spritzenhalter, insbesondere an einer Außenfläche oder einem Außenumfang des Spritzenhalters anliegt und das Eingriffsglied des Spritzenhalters daran hindert, sich quer zur Längsachse von der Längsachse weg zu bewegen. Insbesondere kann der Halteabschnitt zylinderförmig sein und das Eingriffsglied vorzugsweise umgeben. Für die Montage bzw. das Einlegen der Spritze in den Spritzenhalter befindet sich der Spritzenhalter außerhalb des Eingriffs mit dem Halteabschnitt des Gehäuses oder der Nadelschutzhülse. Wenn die Spritze vollständig in den Spritzenhalter eingelegt ist, insbesondere das Eingriffsglied in die Lücke zwischen dem sich verjüngenden Abschnitt und der Nadelschutzkappe eingreift, wird der mit der Spritze aufgenommenen Spritzenhalter in den Eingriff mit dem Halteabschnitt gebracht, so dass verhindert wird, dass sich das Eingriffsglied aus dem Eingriff mit dem verjüngenden Abschnitt quer zur Längsachse, insbesondere von der Längsachse weg oder nach außen, bewegt.

Das Eingriffsglied kann federnd oder elastisch, insbesondere an einem Arm an dem Spritzenhalter gebildet sein, wobei die Spritze über das proximale Ende mit der Nadel voraus in den Spritzenhalter, der vorzugsweise hülsenförmig ist, eingeschoben wird, wobei die Nadelschutzkappe das Eingriffsglied quer zur Längsachse nach außen, d. h. von der Längsachse weg auslenkt, wobei, wenn die Nadelschutzkappe vollständig an dem Eingriffsglied vorbei bewegt wurde, das Eingriffsglied in die Lücke zwischen dem sich verjüngenden Bereich und der Nadelschutzkappe einschnappt. Anschließend wird der Spritzenhalter mit der Spritze in den Eingriff mit dem Halteabschnitt des Gehäuses oder Nadelschutzhülse des Autoinjektors verschoben, wodurch das Eingriffsglied in den Eingriff mit der Lücke zwischen der Nadelschutzkappe und dem sich verjüngenden Abschnitt gehalten wird und aus diesem Eingriff nicht mehr herausfedern kann.

Damit der Benutzer den Autoinjektoren benutzen kann, muss der Benutzer in einem Auslieferungszustands des Autoinjektors eine an dem distalen Ende des Autoinjektors vorgesehene Abziehkappe von dem Gehäuse oder der Nadelschutzhülse abziehen.

Im Auslieferungszustand wird die Nadel von einer Nadelschutzkappe abgedeckt, um die Nadel vor Verschmutzung zu schützen bzw. die Nadel und das Medikament steril zu halten. Die Nadelschutzkappe ist an dem Nadelhalteabschnitt des Spritzenkörpers angeordnet, wobei sich der verjüngende Abschnitt des Spritzenkörpers zwischen dem Nadelhalteabschnitt und dem zylindrischen Abschnitt des Spritzenkörpers befindet. Das Eingriffsglied ist zwischen dem Spritzenkörper, insbesondere dem sich verjüngenden Abschnitt des Spritzenhalters und der Nadelschutzkappe angeordnet, insbesondere so, dass zwischen der Nadelschutzkappe und dem Eingriffsglied ein - wenngleich auch geringer - Spalt entsteht, um zu verhindern, dass das Eingriffsglied eine Kraft auf die Nadelschutzkappe ausübt, wodurch z. B. die Sterilität der Nadel oder des Medikaments gefährdet werden könnte. Die Abziehkappe ist mit dem Gehäuse oder der Nadelschutzhülse lösbar verschnappt, wobei diese Verschnappung gelöst wird, wenn die Abziehkappe von dem Gehäuse oder der Nadelschutzhülse entfernt wird. Die Abziehkappe umfasst einen Schnapphaken, der in die Lücke zwischen dem Spritzenkörper, insbesondere dessen sich verjüngenden Bereichs und dem proximalen Ende der Nadelschutzkappe eingreift. Wenn die Abziehkappe von dem Autoinjektor entfernt wird, hakt der Schnapphaken in das proximale Ende der Nadelschutzkappe ein, wodurch die Nadelschutzkappe von der Spritze gelöst und zusammen mit der Abdeckkappe von dem Autoinjektor entfernt wird. Alternativ kann der Schnapphaken in eine Mantelfläche der Nadelschutzkappe einhaken. In bevorzugten Ausführungsformen ist in dem Auslieferungszustand ebenfalls zwischen der Nadelschutzkappe und dem Schnapphaken ein - wenngleich auch geringer - Spalt vorgesehen, um zu verhindern, dass der Schnapphaken eine Kraft auf die Nadelschutzkappe ausübt, wodurch z. B. die Sterilität der Nadel oder des Medikaments gefährdet werden könnte.

In alternativen Ausführungsformen kann die Abziehkappe anstelle von einem Schnapphaken ein elastisches, schnappbares oder klemmbares Element aufweisen, welches um, an oder in die Nadelschutzkappe greifen kann, um die Nadelschutzkappe von der Spritze abzuziehen oder abzudrehen.

Um die Auslösung der Produktausschüttung zu starten, wird die Nadelschutzhülse relativ zu dem Gehäuse und entlang der Längsachse des Autoinjektors in die proximale Richtung bewegt. Da zwischen der Nadelschutzfeder und der Nadelschutzhülse die Sperrhülse angeordnet, wird die Sperrhülse von der Nadelschutzhülse in die proximale Richtung mitgenommen, wenn die Nadelschutzhülse aus ihrer Ausgangsposition in die proximale Richtung oder in die betätigte Position verschoben wird. Zudem wird die Nadelschutzhülse in die distale Richtung verschoben, wenn die auf Sperrhülse wirkende Nadelschutzfeder die Sperrhülse in die distale Richtung verschiebt. Die Sperrhülse und die Nadelschutzhülse können vorzugsweise als separate Elemente ausgebildet sein. Die Sperrhülse kann mit der Nadelschutzhülse axialfest und drehbar, beispielsweise drehbar verschnappt sein oder die Sperrhülse und die Nadelschutzhülse können lose aneinanderliegen.

Das Gehäuse oder der Mechanikhalter kann eine Eingriffsnocke aufweisen, welche vor dem Auslösen der Produktausschüttung in das Vortriebsglied eingreift, wodurch das Vortriebsglied daran gehindert wird, sich relativ zu dem Gehäuse oder dem Mechanikhalter in die Ausschüttrichtung zu bewegen. Die Ausschüttfeder ist vorgespannt in dem Vortriebsglied gehalten, wenn die Eingriffsnocke des Gehäuses oder des Mechanikhalters in dem Eingriff oder in der Kopplung mit dem Vortriebsglied ist. Die Eingriffsnocke kann an einem federnden oder elastischen Arm des Gehäuses oder des Mechanikhalters vorgesehen sein. Alternativ kann die Eingriffsnocke federnd oder elastisch aufgebildet sein. Der Eingriff oder die Kopplung der Eingriffsnocke in das Vortriebsglied ist für die Produktausschüttung lösbar. Wenn der Eingriff oder die Kopplung gelöst ist, ist das Vortriebsglied für die Bewegung in die Ausschüttrichtung freigegeben. Die Ausschüttfeder kann das Vortriebsglied relativ zu dem Gehäuse oder dem Mechanikhalter um den Ausschütthub in die Ausschüttrichtung verschieben. Die Ausschüttfeder kann sich entspannen. Das Vortriebsglied kann eine Ausnehmung für die Eingriffsnocke des Gehäuses oder des Mechanikhalters aufweisen, wobei diese Kopplung zwischen dem Vortriebsglied und dem Gehäuse oder dem Mechanikhalter gelöst ist, wenn das Gehäuse oder Mechanikhalter, insbesondere die Eingriffsnocke, aus dem Eingriff oder aus der Kopplung mit dem Vortriebsglied, insbesondere aus der Ausnehmung des Vortriebsglieds ausgerückt ist. Insbesondere kann die Eingriffsnocke dadurch aus dem Eingriff oder aus der Kopplung mit dem Vortriebsglied gelöst werden, dass die Nadelschutzhülse aus der Ausgangsposition um den Betätigungshub in die betätigte Position verschoben wird. Z. B. kann die Eingriffsnocke von der Nadelschutzhülse oder der Sperrhülse in dem axialfesten Eingriff oder in der axialfesten Kopplung mit dem Vortriebsglied gehalten werden, wenn die Nadelschutzhülse nicht in ihrer betätigten Position oder in ihrer Ausgangsposition ist. Z. B. kann ein Innenumfang der Nadelschutzhülse oder ein Innenumfang der Sperrhülse die Eingriffsnocke in dem Eingriff oder in der Kopplung mit dem Vortriebsglied halten, wobei die Eingriffsnocke an dem Innenumfang der Nadelschutzhülse oder an dem Innenumfang der Sperrhülse anliegen kann.

Durch das Verschieben der Nadelschutzhülse in ihre betätigte Position kann die Nadelschutzhülse oder die Sperrhülse der Eingriffsnocke des Gehäuses oder des Mechanikhalters erlauben, insbesondere mit einer Bewegung quer zu der Längsachse des Autoinjektors aus dem Eingriff oder aus der Kopplung mit dem Vortriebsglied auszurücken. Beispielsweise kann eine Aussparung an der Nadelschutzhülse oder an der Sperrhülse vorgesehen sein, welche derart axial an der Nadelschutzhülse oder an der Sperrhülse positioniert ist, dass in der betätigten Position der Nadelschutzhülse die Eingriffsnocke aus dem Eingriff oder aus der Kopplung mit dem Vortriebsglied und/oder quer zu der Längsachse in die Aussparung ausrücken kann.

Um ein Zurückschieben der Nadelschutzhülse nach erfolgter Produkthausschüttung zu verhindern, so dass zumindest die Nadelspitze der Nadel nicht aus dem distalen Ende der Nadelschutzhülse hervortritt, kann vorzugsweise an der Sperrhülse und an einem gehäusefesten Element, insbesondere dem Mechanikhalter, dem Gehäuse oder der Verschlusskappe die Verriegelungseinrichtung des Autoinjektors vorgesehen sein.

Besonders bevorzugt ist die Verriegelungseinrichtung des Autoinjektors an der Sperrhülse und dem Mechanikhalter vorgesehen. Dazu kann die Sperrhülse das federnd oder elastisch angeordnete Verriegelungsglied umfassen und der Mechanikhalter kann den korrespondierenden Verriegelungsanschlag aufweisen. Alternativ kann das federnd oder elastisch angeordnete Verriegelungsglied an dem Mechanikhalter vorgesehen sein und der korrespondierende Verriegelungsanschlag an der Sperrhülse angeordnet sein.

In der Ausgangsposition oder dem Auslieferungszustand ist die Sperrhülse relativ zu dem Mechanikhalter drehfest angeordnet. In bevorzugten Ausführungsformen kann an der Sperrhülse und an dem Mechanikhalter eine lösbare Rotationssicherung vorgesehen sein. Alternativ kann die Rotationssicherung an dem Gehäuse oder an der Verschlusskappe und der Sperrhülse vorgesehen sein. Die Rotationssicherung kann als Eingriff oder als Kopplung zwischen einer Rippe und einer ersten Nut ausgebildet sein. Die Rotationssicherung dient in der Ausgangsposition oder in dem Auslieferungszustand der Verhinderung einer Rotation zwischen der Sperrhülse und dem Mechanikhalter. Der Mechanikhalter kann die Rippe und die Sperrhülse die erste Nut aufweisen. Alternativ kann die Rippe an der Sperrhülse und die erste Nut an dem Mechanikhalter vorgesehen sein.

Da die Sperrhülse und die Nadelschutzhülse axialfest miteinander verbunden sind oder lose aneinanderliegen, kann durch das Verschieben der Nadelschutzhülse in ihre betätigte Position die Sperrhülse ebenfalls in die proximale Richtung bewegt werden. Dabei wird die Rotationssicherung zwischen der Sperrhülse und dem Mechanikhalter gelöst, da die erste Nut der Sperrhülse und die Rippe aus dem Eingriff oder aus der Kopplung gelangen. Wenn die Rotationssicherung zwischen der Sperrhülse und dem Mechanikhalter gelöst ist, kann die Sperrhülse relativ zu dem Mechanikhalter drehen. Die Rotationssicherung ist zumindest in der betätigen Position der Nadelschutzhülse gelöst. Durch das Verschieben der Nadelschutzhülse in ihre betätigte Position kann zudem die Eingriffsnocke des Mechanikhalters oder des Gehäuses aus dem Eingriff oder aus der Kopplung mit der Ausnehmung der Vortriebglieds gelangen, so dass das Vortriebsglied aufgrund der vorgespannten Kraft der Ausschüttfeder in die distale Richtung bewegt werden kann, um das Produkt aus dem Produktbehälter, insbesondere der Spritze auszuschütten. Besonders bevorzugt kann die Sperrhülse die Aussparung aufweisen, so dass das Eingriffsnocke radial nach aussen bewegbar ist, um aus dem Eingriff oder aus der Kopplung mit dem Vortriebsglied zu gelangen. Besonders bevorzugt ist die Eingriffsnocke an dem federnden oder elastischen Arm angeordnet oder federnd oder elastisch ausgebildet sein, um quer zur Längsachse des Autoinjektors und/oder radial nach aussen bewegbar zu sein.

In bevorzugten Ausführungsformen können die Sperrhülse und der Mechanikhalter zudem in einen Eingriff oder in einer Kopplung gelangen, wobei dieser Eingriff oder diese Kopplung dazu ausgelegt, dass die Sperrhülse relativ zu dem Mechanikhalter dreht, wenn die Nadelschutzhülse relativ zu dem Mechanikhalter in die Nadelschutzposition verschoben wird, wobei in der Nadelschutzposition eine Verriegelung zwischen der Sperrhülse und dem Mechanikhalter die Nadelschutzhülse so relativ zu dem Mechanikhalter gegen ein Zurückschieben in die proximale Richtung verriegelt, dass die die Nadelspitze nicht aus dem distalen Ende der Nadelschutzhülse hervortreten kann. Alternativ kann dieser Eingriff oder diese Kopplung zwischen dem Gehäuse oder der Verschlusskappe und der Sperrhülse vorgesehen sein.

Dazu kann der federnde oder elastische Arm oder die federnde oder elastische Eingriffsnocke eine Führungsnocke aufweisen. Die Eingriffsnocke oder der Arm der Eingriffsnocke kann die Führungsnocke aufweisen, welche durch die Ausrückbewegung der Eingriffsnocke aus dem Vortriebsglied in den Eingriff oder in die Kopplung mit einer Führungskulisse an der Sperrhülse gelangen kann. Die Führungsnocke kann quer zur Längsachse des Autoinjektors und/oder radial nach aussen bewegbar angeordnet sein. Die Führungsnocke kann z. B. an dem federnden oder elastischen Arm, an dem der Eingriffsnocke angeordnet ist, sein. Der Eingriff oder die Kopplung zwischen der Führungsnocke und der Führungskulisse kann eine Kulissensteuerung, insbesondere eine Rotationsführung bilden. Die Eingriffsnocke und die Führungsnocke können so aufeinander abgestimmt sein, dass die Kulissensteuerung, insbesondere die Rotationsführung zwischen der Sperrhülse und dem Mechanikhalter oder dem Gehäuse bereits im Eingriff oder gekoppelt ist, wenn die Eingriffsnocke noch nicht vollständig aus dem Eingriff mit dem Vortriebsglied gelöst ist. Die Eingriffsnocke und die Führungsnocke können so aufeinander abgestimmt sein, dass die Führungsnocke bereits in die Führungskulisse der Sperrhülse eingreift oder koppelt, wenn die Eingriffsnocke noch nicht vollständig aus dem Eingriff mit dem Vortriebsglied gelöst ist. Hierdurch wird vorteilhaft erreicht, dass zuerst der Eingriff oder die Kopplung zwischen der Sperrhülse und dem Gehäuse oder dem Mechanikhalter hergestellt ist, bevor die axialfeste Verbindung zwischen dem Gehäuse oder dem Mechanikhalter und dem Vortriebsglied gelöst und damit ein erneutes oder weiteres Zurückschieben der Nadelschutzhülse gesperrt wird. Insbesondere wenn die Führungsnocke in seiner Führungskulisse ist, kann sich das Vortriebsglied relativ zu dem Gehäuse oder zu dem Mechanikhalter in distale Richtung bewegen, insbesondere aufgrund der in der Ausschüttfeder gespeicherten Energie. Das Vortriebsglied kann die Führungsnocke daran hindern, aus dem Eingriff oder aus der Kopplung mit der Sperrhülse auszurücken, wenn sich das Vortriebsglied relativ zu dem Gehäuse oder zu dem Mechanikhalter in die distale Richtung bewegt. Dies gilt vorzugsweise auch am Ende des Ausschütthubs. Das Vortriebsglied hält oder drückt die Führungsnocke, insbesondere die Führungsnocke des Gehäuses oder des Mechanikhalters in die Führungskulisse, insbesondere in die Führungskulisse der Sperrhülse während und am Ende des Ausschütthubs.

Vorzugsweise sind die Eingriffsnocke und die Führungsnocke an einem gemeinsamen federnden oder elastischen Arm gebildet, wobei die Eingriffsnocke, z. B. radial, zu der Längsachse hin und die Führungsnocke, z. B. radial, von der Längsachse wegweisen. Die Eingriffsnocke und die Führungsnocke können, vorzugsweise radial zwischen dem Vortriebsglied und der Nadelschutzhülse oder der Sperrhülse angeordnet sein.

Die Führungsnocke kann durch eine radiale Auslenkung der Eingriffsnocke nach aussen in den Eingriff oder in die Kopplung mit einer an der Sperrhülse vorgesehenen Führungskulisse gelangen. Der Eingriff oder die Kopplung zwischen der Führungsnocke und der Führungskulisse ist als Kulissensteuerung, insbesondere als Rotationsführung ausgebildet. Am Ende der Produktausschüttung kann der Patient den Autoinjektor von der Einstichstelle wegnehmen. Dabei bewegt sich die Nadelschutzhülse und die Sperrhülse in die distale Richtung. Aufgrund des Eingriffs zwischen der Führungsnocke des Gehäuses oder des Mechanikhalters und der Führungskulisse der Sperrhülse kann sich während der axialen Bewegung der Sperrhülse in die distale Richtung die Sperrhülse relativ zu dem Gehäuse oder relativ zu dem Mechanikhalter drehen. Dabei wird die Eingriffsnocke durch das Vortriebsglied in dem Eingriff oder in der Kopplung mit der Führungskulisse gehalten. Während oder nach der Drehung der Sperrhülse relativ zu dem Gehäuse oder relativ zu dem Mechanikhalter kann die Führungsnocke in die axiale Flucht zu der ersten Nut gelangen. Zudem kann während oder nach der Drehung der Sperrhülse relativ zu dem Gehäuse oder relativ zu dem Mechanikhalter die Rippe in die axiale Flucht mit einer zweiten Nut gelangen. Die zweite Nut kann an der Sperrhülse, dem Gehäuse oder Mechanikhalter vorgesehen sein. Durch den Eingriff der Rippe in die zweite Nut kann die Nadelschutzhülse relativ zu dem Gehäuse aus ihrer betätigten Position um den Nadelschutzhub in die Nadelschutzposition verschoben werden, insbesondere mittels der Nadelschutzfeder gespeicherten Federenergie. Durch die relative Drehung zwischen der Sperrhülse und dem Mechanikhalter oder dem Gehäuse gelangt ferner das federnd oder elastisch angeordnete Verriegelungsglied und der korrespondierende Verriegelungsanschlag in eine axiale Flucht. Während der Bewegung der Nadelschutzhülse und der Sperrhülse in die distale Richtung, insbesondere in die Nadelschutzposition gelangen das federnd oder elastisch angeordnete Verriegelungsglied und der korrespondierende Verriegelungsanschlag in eine Verriegelungsposition. Bei einer erneuten Bewegung der Nadelschutzhülse und der Sperrhülse in die proximale Richtung gelangen das federnd oder elastisch angeordnete Verriegelungsglied und der korrespondierende Verriegelungsanschlag in Anschlagkontakt, insbesondere in axialen Anschlagkontakt, sodass eine weitere Bewegung der Nadelschutzhülse und der Sperrhülse relativ zu dem Gehäuse in die proximale Richtung verhindert wird.

Alternativ kann die Führungsnocke an der Sperrhülse und die Führungskulisse an dem Mechanikhalter vorgesehen sein.

In bevorzugten Ausführungsformen kann das Vortriebsglied ein oder mehrere Signalglieder aufweisen. Das Signalglied kann an einem federnden oder elastischen Arm des Vortriebsglieds vorgesehen sein oder federnd oder elastisch ausgebildet sein. Das Signalglied kann als ein radial nach aussen ragender Vorsprung ausgebildet sein. Der Mechanikhalter, die Verschlusskappe oder das Gehäuse kann entlang der Längsachse des Autoinjektors mehrere Öffnungen aufweisen. Diese Öffnungen sind gleichmässig oder ungleichmässig voneinander beabstandet entlang der Längsachse des Autoinjektors angeordnet. Das Signalglied ist vorzugsweise an einem proximalen Ende oder an einem proximalen Bereich des Vortriebsglied vorgesehen. Falls mehrere Signalglieder vorgesehen sind, können die Signalglieder entlang der Längsachse des Vortriebsglieds oder in Umgangsrichtung des Vortriebsglieds gleichmässig oder ungleichmässig voneinander beabstandet an dem Vortriebsglied angeordnet sein. Die Positionierung des Signalglieds und die Positionierung der Öffnungen entlang der Längsachse des Autoinjektors sind derart miteinander abgestimmt, dass das Signalglied während dem ganzen und zumindest teilweisen Ausschütthub des Kolbens oder des Vortriebsglieds mit den Öffnungen des Mechanikhalters, der Verschlusskappe oder des Gehäuses derart zusammenwirken, dass ein Ausschüttsignal, insbesondere ein akustisches, haptisches und/oder visuelles Signal erzeugt wird. Somit kann sichergestellt werden, dass während dem ganzen oder zumindest teilweisen Ausschütthub des Kolbens oder des Vortriebsglieds dem Patienten angezeigt werden kann, dass der Ausschüttvorgang im Gange ist oder von statten geht. Das Ausschüttsignal kann während dem Ausschüttvorgang gleichmässig, beschleunigt oder verzögert erzeugt werden. Das Ausschüttsignal kann als Rattern oder Klicken ausgebildet sein, insbesondere als ein wiederholtes Rattern oder Klicken ausgebildet sein. Alternativ oder zusätzlich kann das Gehäuse und/oder der Mechanikhalter ein Sichtfenster aufweisen, um die Bewegung bzw. die Position, insbesondere die fortlaufende Bewegung bzw. die fortlaufende Position des Signalglieds zu erkennen. Alternativ oder zusätzlich kann während der Produktausschüttung der Autoinjektor vibrieren. Zudem kann der Autoinjektor eine Verstärkungseinrichtung, beispielsweise einen Resonanzkörper, ein Exzentriker oder ein Vergrösserungsglas umfassen, um das Ausschüttsignal zu verstärken. Zudem kann die Verwendung von mehreren Signalgliedern das Ausschüttsignal verstärken.

In alternativen Ausführungsformen kann das Vortriebsglied mehrere Öffnungen aufweisen. Diese Öffnung können gleichmässig oder ungleichmässig voneinander beabstandet entlang der Längsachse des Vortriebsglieds angeordnet sein. Das Gehäuse, die Verschlusskappe oder der Mechanikhalter kann ein oder mehreren Signalglieder aufweisen. Das Signalglied kann an einem federnden oder elastischen Arm des Gehäuses, der Verschlusskappe oder des Mechanikhalters angeordnet sein. Das Signalglied kann als ein radial nach innen ragender Vorsprung ausgebildet sein. Die Ausgestaltung, Anordnung und Wirkung der Öffnungen und des Signalglieds entsprechen ansonsten der bereits oben erwähnten Ausführungsformen. Somit kann sichergestellt werden, dass während dem ganzen oder zumindest teilweisen Ausschütthub des Kolbens oder des Vortriebsglieds dem Patienten angezeigt werden kann, dass der Ausschüttvorgang durchgeführt wird.

In alternativen Ausführungsformen kann das Gehäuse, die Verschlusskappe oder der Mechanikhalter ein oder mehrere Signalglieder aufweisen. Das Signalglied kann an einem federnden oder elastischen Arm des Gehäuses, der Verschlusskappe oder des Mechanikhalters angeordnet sein. Das Signalglied kann als ein radial nach innen ragender Vorsprung ausgebildet sein. Falls mehrere Signalglieder vorgesehen sind, können die Signalglieder entlang der Längsachse oder alternativ in Umfangsrichtung des Gehäuses, der Verschlusskappe oder des Mechanikhalters gleichmässig oder ungleichmässig voneinander beabstandet an dem Gehäuse, der Verschlusskappe oder des Mechanikhalters angeordnet sein. Das Signalglied kann während dem Ausschüttvorgang über die Ausschüttfeder, insbesondere über die Windungen der Ausschüttfeder gleiten, um ein akustisches, haptisches und/oder visuelles Signal zu erzeugen. Somit kann sichergestellt werden, dass während dem ganzen oder zumindest teilweisen Ausschütthub des Kolbens oder des Vortriebsglieds dem Patienten angezeigt werden kann, dass der Ausschüttvorgang erfolgt. Dazu kann das Vortriebsglied entlang der Längsachse des Vortriebsglieds eine Öffnung, insbesondere eine Längsöffnung aufweisen, wobei das oder die Signalglieder durch die Öffnung, insbesondere der Längsöffnung des Vortriebsglied ragen können, um mit der Ausschüttfeder, insbesondere mit den Windungen der Ausschüttfeder derart zusammen zu wirken, dass ein Ausschüttsignal erzeugt wird. Die Ausschüttfeder kann somit innerhalb des Vortriebsglieds angeordnet sein. In alternativen Ausführungsformen kann die Ausschüttfeder nicht innerhalb des Vortriebsglieds sondern ausserhalb des Vortriebsglieds angeordnet sein. Falls die Ausschüttfeder ausserhalb des Vortriebsglieds angeordnet ist, kann das Vortriebsglieds als Führung der Ausschüttfeder dienen, so dass die Ausschüttfeder vorgespannt und/oder entspannt geführt oder gestützt ist. Somit kann auf eine Führung, welche an dem Gehäuse, dem gehäusefesten Element, insbesondere der Verschlusskappe angeordnet ist, verzichtet werden. Zudem kann der Autoinjektor eine Verstärkungseinrichtung, beispielsweise einen Resonanzkörper, Exzentriker oder Vergrösserungsglas umfassen, um das Ausschüttsignal zu verstärken. Zudem kann die Verwendung von mehreren Signalglieder das Ausschüttsignals verstärken.

Die Erfindung und weitere Aspekte zur Erfindung wurden anhand mehrerer bevorzugter Ausführungsformen beschrieben. Im Folgenden werden besonders bevorzugte Ausführungsformen anhand von Figuren beschrieben. Die dabei offenbarten Merkmale bilden den Gegenstand der Erfindung einzeln und in jeglicher Merkmalskombination vorteilhaft weiter. Es zeigen:
- Figur 1: Explosionsdarstellung eines Autoinjektors gemäß einer besonders bevorzugten Ausführungsform,
- Figuren 2a, 2b: die Vorrichtung aus Figur 1 in einem Auslieferungszustand, wobei die Figuren 2a und 2b durch die Längsachse der Vorrichtung verlaufende Schnittansichten sind, wobei die Schnittansichten um die Längsachse um 90° winkelversetzt sind,
- Figuren 3a, 3b: die Vorrichtung und die Ansichten aus den Figuren 2a und 2b, wobei sich eine Nadelschutzhülse (3) in ihrer betätigten Position befindet und ein axialfester Eingriff oder eine axialfeste Kopplung zwischen einem Vortriebsglied (6) und dem Mechanikhalter (5) lösbar ist.
- Figuren 4a, 4b: die Vorrichtung und die Ansichten aus den Figuren 2a und 2b, wobei ein Ausschüttsignal während der Produktausschüttung erzeugt wird.
- Figuren 5a, 5b: die Vorrichtung und die Ansichten aus den Figuren 2a und 2b, wobei sich die Vorrichtung in der ausgeschütteten Position befindet, wobei kein Ausschüttsignal erzeugt wird oder das Ausschüttsignal verstummt ist.
- Figuren 6a, 6b: die Vorrichtung und die Ansichten aus den Figuren 2a und 2b, wobei die Nadelschutzhülse in ihrer Nadelschutzposition ist.
- Figur 7a, 7b: eine alternative Vorrichtung mit einer alternativen Signaleinrichtung, wobei die Figuren 7a und 7b durch die Längsachse der Vorrichtung verlaufende Schnittansichten sind und wobei ein Ausschüttsignal während der Produktausschüttung erzeugt wird.
- Figur 8a, 8b: eine weitere alternative Vorrichtung mit einer weiteren alternativen Signaleinrichtung, wobei die Figuren 8a und 8b durch die Längsachse der Vorrichtung verlaufende Schnittansichten sind und wobei ein Ausschüttsignal während der Produktausschüttung erzeugt wird.
- Figur 9a, 9b: eine weitere alternative Vorrichtung mit einem Adapter, um den Abstand zwischen einem Vortriebsglied (6") und einem Kolben einzustellen, wobei die Vorrichtung in der Figur 9a vor der Einstellung und die Vorrichtung in der Figur 9b nach der Einstellung dargestellt ist.
- Figur 10a, 10b: eine weitere alternative Vorrichtung mit einem Adapter, beim Start der Produktausschüttung die kinetische Energie eines Vortriebsglied (6‴) zumindest teilweise zu absorbieren, wobei die Vorrichtung in der Figur 10a vor der Absorption und die Vorrichtung in der Figur 10b nach der Absorption dargestellt ist.
- Figur 11a, 11b: eine weitere alternative Vorrichtung mit einem Adapter, beim Start der Produktausschüttung die kinetische Energie eines Vortriebsglied (6‴) zumindest teilweise zu absorbieren, wobei die Vorrichtung in der Figur 11a vor der Absorption und die Vorrichtung in der Figur 11b nach der Absorption dargestellt ist.

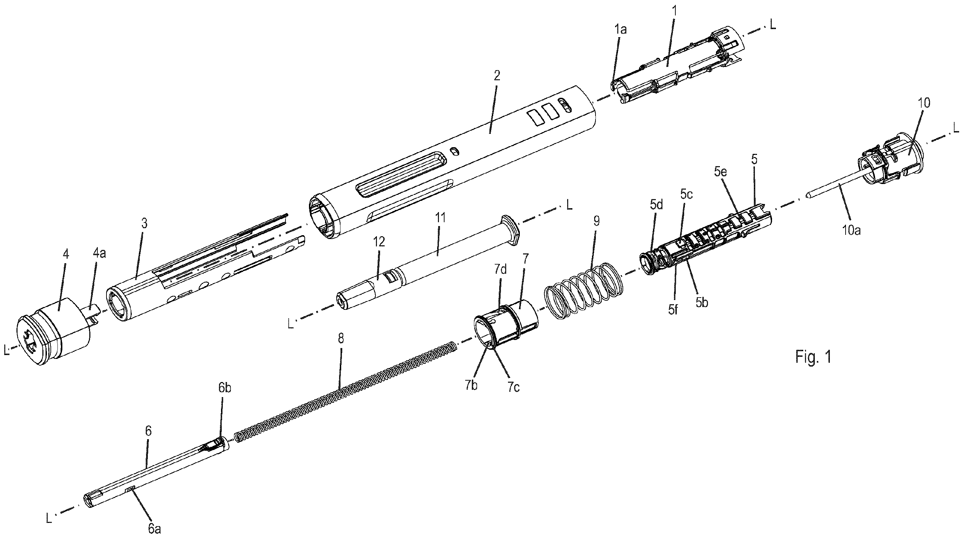

Bezugnehmend auf die Figuren 1 bis 11b werden nun die strukturellen Merkmale und die Funktionen bevorzugten Ausführungsformen beschrieben.

Der Autoinjektor weist ein hülsenförmiges, längliches Gehäuse (2) mit einer Längsachse L auf, welches an seinem proximalen Ende eine Verschlusskappe (10) aufweist, die formschlüssig mit dem Gehäuse (2) dreh- und axialfest verbunden ist. Die Verschlusskappe (10) ist mit dem Gehäuse (2) beispielsweise über eine Rastverbindung verbunden.

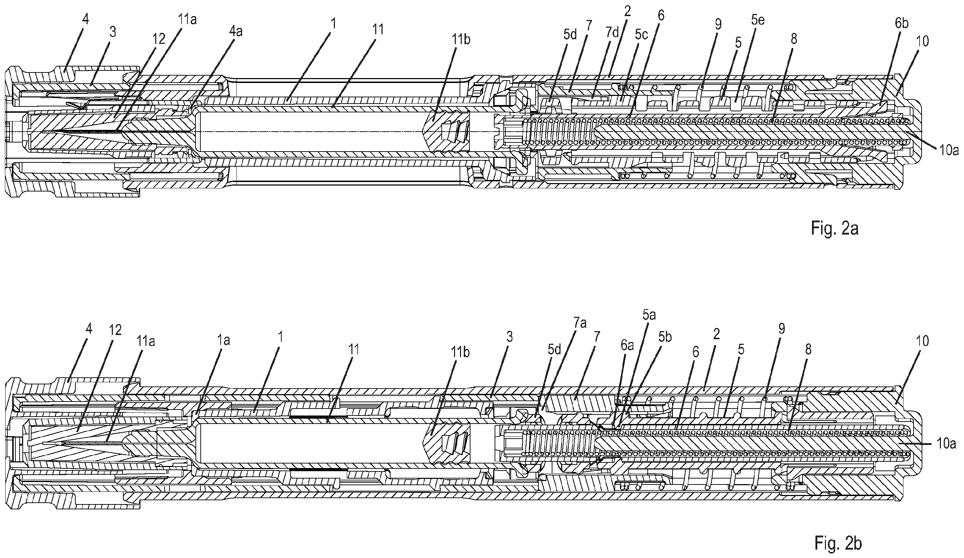

In dem Auslieferungszustand (Figuren 2a und 2b) ist an dem distalen Ende des Autoinjektors eine Abziehkappe (4) angeordnet, die vor der Verwendung des Autoinjektors abgezogen oder abgedreht und entfernt wird.

In dem Gehäuse (2) ist ein Produktbehälter (11) in der Gestalt einer Spritze (11) in Bezug auf das Gehäuse (2) - abgesehen von der Montage des Autoinjektors - entlang der Längsachse L unverschiebbar aufgenommen. Der Produktbehälter (11) weist einen hülsenförmigen Spritzenkörper auf, der einen Kolben (11b), der dichtend an dem Innenumfang des Spritzenkörpers anliegt, umgibt. Der Spritzenkörper weist an seinem distalen Ende eine insbesondere unlösbar mit dem Spritzenkörper verbundene Nadel (11a) auf, deren distales Ende von der Nadelspitze gebildet wird. Zwischen der Nadel (11a) und dem Kolben (11b) ist ein flüssiges Produkt, insbesondere ein Medikament, innerhalb des Spritzenkörpers angeordnet, wobei das flüssige Produkt durch Verschieben des Kolbens (11b) in eine Ausschüttrichtung, d. h. in distale Richtung oder zu der Nadel (11a) hin das Produkt durch die hohle Nadel (11a) aus der Spritze (11) ausschüttet. Der Spritzenkörper weist an seinem proximalen Ende einen sogenannten Fingerflansch auf, der radial nach außen über den Außenumfang des zylindrischen Spritzenkörpers ragt.

Der Produktbehälter (11) ist in einem Produktbehälterhalter (1), der als Spritzenhalter (1) bezeichnet wird, so aufgenommen, dass er zumindest gegen eine Bewegung entlang der Längsachse L in distale Richtung relativ zu dem Spritzenhalter (1) gesichert ist. Der Spritzenhalter ist besonders bevorzugt axial- und drehfest mit dem Gehäuse (2) verbunden.. Der Spritzenhalter (1) weist mindestens eine nach innen ragende Schulter (1a) auf, an der sich ein verjüngender Abschnitt der Spritze (11), der distal des zylindrischen Spritzenkörperabschnitts, welcher den Kolben (11b) führt, abstützt.

Um zu verhindern, dass die Spritze (11) relativ zu dem Spritzenhalter (1) in die proximale Richtung verschiebbar ist, wird die Spritze (11) an seinem proximalen Ende durch einen auf den Spritzenkörper wirkenden Halter in den Eingriff mit der Schulter (1a) gedrückt. Der Halter ist als eine Haltefeder (5d) ausgebildet, wobei die Haltefeder (5d) an einem Mechanikhalter (5) vorgesehen ist. Der Mechanikhalter (5) ist in Bezug auf das Gehäuse (2) entlang der Längsachse L axial- und drehfest angeordnet. Der hülsenförmige Mechanikhalter (5) kann mit dem Gehäuse (2) verschnappt oder verrastet sein. Durch die Haltefeder (5d) können Längenunterschiede der Spritze (11), die aufgrund von Herstellungstoleranzen entstehen können, ausgeglichen werden, wobei der feste Sitz der Spritze (11) an der Schulter (1a) sichergestellt ist.

Die Spritze (11) ist in Bezug auf das Gehäuse (2) so angeordnet, dass die Nadelspitze distal über das distale Ende des Gehäuses (2) übersteht. Im Auslieferungszustand (Figuren 2a und 2b) des Autoinjektors, d. h., wenn die Abziehkappe (4) an dem Autoinjektor angeordnet ist, wird die Nadel (11a) von einer Nadelschutzkappe (12) abgedeckt, um die Nadel (11a) vor Verschmutzung zu schützen bzw. die Nadel (11a) und das Medikament steril zu halten. Die Nadelschutzkappe (12) ist an einem Nadelhalteabschnitt des Spritzenkörpers angeordnet, wobei sich der verjüngende Abschnitt des Spritzenkörpers zwischen dem Nadelhalteabschnitt und dem zylindrischen Abschnitt des Spritzenkörpers befindet. Die Schulter (1a) ist zwischen dem Spritzenkörper, insbesondere dem verjüngenden Abschnitt des Spritzenkörpers und dem proximalen Ende der Nadelschutzkappe (12) angeordnet. Die Abziehkappe (4) ist mit dem Gehäuse (2) oder einer Nadelschutzhülse (3) lösbar verschnappt, wobei diese Verschnappung gelöst wird, wenn die Abziehkappe (4) von dem Gehäuse (2) oder der Nadelschutzhülse (3) entfernt wird. Die Abziehkappe (4) weist einen Schnapphaken (4a), der an das proximale Ende der Nadelschutzkappe (12) angreifen kann. Wenn die Abziehkappe (4) von dem Autoinjektor entfernt wird, hakt der Schnapphaken (4a) an das proximale Ende der Nadelschutzkappe (12), wodurch die Nadelschutzkappe (12) von der Spritze (11) gelöst und zusammen mit der Abdeckkappe (4) von dem Autoinjektor entfernt wird.

Der Autoinjektor weist eine Nadelschutzhülse (3) auf, die relativ zu dem Gehäuse (2) und entlang der Längsachse L um einen Betätigungshub in die proximale Richtung in eine betätigte Position verschiebbar ist, um eine Produktausschüttung auszulösen. In der Ausgangsposition der Nadelschutzhülse (3) steht das distale Ende der Nadelschutzhülse (3) distal über die Nadelspitze der Nadel (11a) über, so dass ein Zugriff auf die Nadelspitze zunächst verhindert wird. Durch Verschieben der Nadelschutzhülse (3) um den Betätigungshub wird die Nadelschutzhülse (3) soweit in die proximale Richtung verschoben, dass die Nadel (11a) aus dem distalen Ende der Nadelschutzhülse (3) hervortritt, insbesondere mit einer Länge hervortritt, welche der Injektionstiefe der Nadel in die Einstichstelle entspricht. Bevorzugt soll die Nadel (11a) soweit über das distale Ende der Nadelschutzhülse (3) überstehen, dass eine subkutane Injektion erfolgen kann.

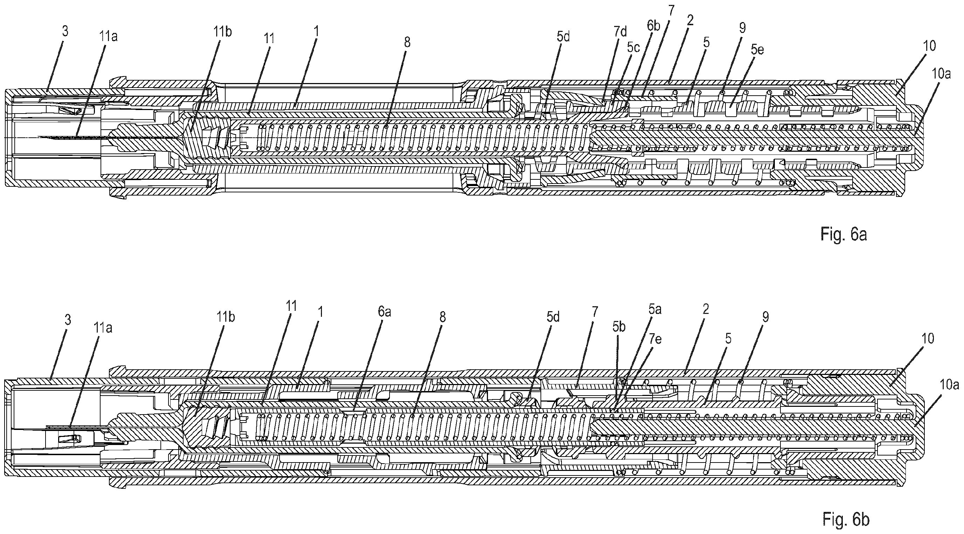

Nach der erfolgten Injektion kann die Nadelschutzhülse (3) relativ zu dem Gehäuse (2) aus der betätigten Position entlang der Längsachse L um einen Nadelschutzhub in die distale Richtung in eine Nadelschutzposition (Figuren 6a und 6b) verschoben werden. In der Nadelschutzposition steht das distale Ende der Nadelschutzhülse (3) distal über die Nadelspitze über, so dass ein Zugriff auf die Nadelspitze verhindert und eine Verletzungsgefahr verringert wird. Die Nadelschutzhülse (3) kann, wie weiter unten beschrieben wird, insbesondere mittels Verriegelungseinrichtung gegen erneutes Zurückschieben aus der Nadelschutzposition blockiert werden.

Der Autoinjektor weist ferner ein hülsenförmiges Vortriebsglied (6). Eine Ausschüttfeder (8) ist innerhalb des hülsenförmigen Vortriebsglieds (6) angeordnet. Die Ausschüttfeder (8) ist in dem Ausgangszustand vorgespannt in dem Vortriebsglied (6) angeordnet und ist mit so viel Energie vorgespannt, dass sie das in der Spritze (11) enthaltene Produkt, insbesondere vollständig durch Verschieben des Vortriebsglieds (6) um einen Ausschütthub aus der Spritze (11) ausschütten kann.

Im Auslieferungszustand oder in der Ausgangsposition des Autoinjektors besteht zwischen dem Kolben (11b) und dem distalen Ende des Vortriebsglieds (6) ein Abstand, so dass das Vortriebsglied (6) erst während der Ausführung des Ausschütthubs an den Kolben (11b) anschlägt und diesen in die Ausschüttrichtung mitnimmt.

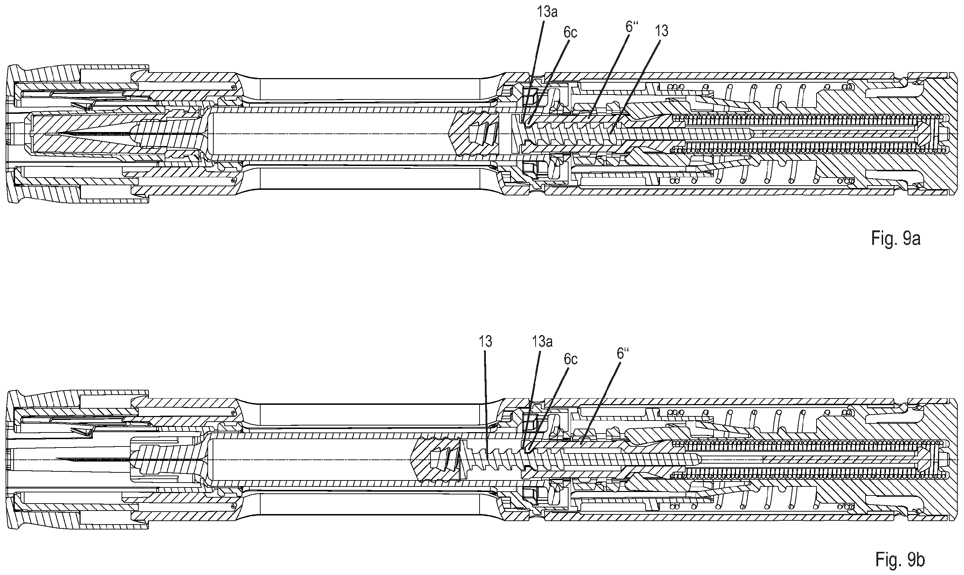

In bevorzugten Ausführungsformen, wie in den Figuren 9a und 9b gezeigt, kann bei der Montage des Autoinjektors der Abstand zwischen dem Vortriebsglied (6") und dem Kolben angepasst, insbesondere verkleinert werden, indem ein Adapter (13) verwendet wird. Dazu kann der Adapter (13) eine Zahnung (13a) aufweisen, welche mit einer an dem Vortriebsglied (6") angeordneten Gegenzahnung (6c) in einem Eingriff ist. Durch eine axiale Relativbewegung zwischen dem Adapter (13) und dem Vortriebsglied (6") kann der Abstand zwischen dem in der Spritze vorgesehenen Kolben und dem Adapter (13) eingestellt werden kann, da aufgrund der verschiedenen Füllvolumina der Spritzen der Kolben an unterschiedlicher Stelle innerhalb der Spritze angeordnet ist.

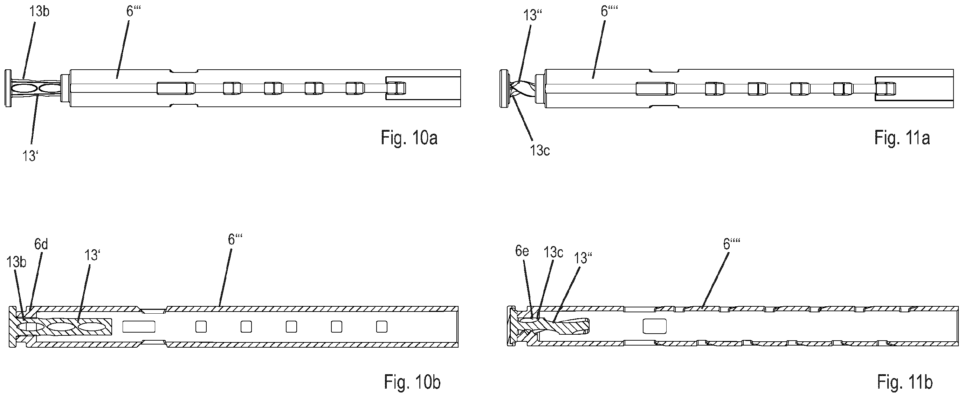

Wie in den Figuren 10a, 10b, 11a und 11b gezeigt ist, kann die Verwendung eines Adapters (13'; 13") in einem Autoinjektor zudem oder alternativ während dem Start der Ausschüttung die Gefahr von Glasbruch verhindert. Diese Gefahr tritt insbesondere auf, wenn beim Start der Ausschüttung das Vortriebsglied (6‴; 6ʺʺ) mit einer schlagartig auftretenden Aufprallenergie auf den Kolben auftritt. Diese Energie wird durch die Reibung zwischen dem Adapter (13'; 13") und dem Spritzenkörper zumindest teilweise absorbiert, wenn der Adapter (13'; 13") auf den im Innern der Spritze angeordneten Kolben auftrifft. Dazu (Figuren 10a und 10b) kann der Adapter (13') ein Quetschelement (13b) aufweisen, welches mit einem an dem Vortriebsglied (6‴) vorgesehenen Quetschgegenelement (6d) zusammenwirken kann, so dass die Aufprallenergie aufgrund einer Quetschung des Quetschelements (13b) zumindest teilweise absorbiert wird. Alternativ (Figuren 11a und 11b) kann der Dämpfungseffekt durch eine Gewindeverbindung (13c, 6e) zwischen einem Adapter (13') und einem Vortriebsglied (6ʺʺ), insbesondere zwischen einem Gewinde (13c) an dem Adapter (13') und einem Gegengewinde (6e) an dem Vortriebsglied (6"") erzielt werden.

Der Mechanikhalter (5) umfasst eine Eingriffsnocke (5a) und eine Führungsnocke (5b). In diesem Beispiel umfasst der Mechanikhalter (5) einen federnden oder elastischen Arm, welcher die Eingriffsnocke (5a) und die Führungsnocke (5b) umfasst. Die Eingriffsnocke (5a) weist radial zu der Längsachse L hin, wobei die Führungsnocke (5b) radial von der Längsachse L weg weist. Die Eingriffsnocke (5a) greift in eine Ausnehmung (6a), die an dem Vortriebselement (6) vorgesehen ist, ein, wodurch eine Bewegung des Vortriebsglieds (6) relativ zu dem Mechanikhalter (5) in die distale Richtung oder in die Ausschüttrichtung verhindert wird. Hierdurch wird die Ausschüttfeder (8) in ihrem gespannten Zustand gehalten. Die Verschlusskappe (10) weist eine Führung (10a) auf, der durch das proximale Ende der Ausschüttfeder (8) in die Seele der Ausschüttfeder (8) eingefügt ist. Die Führung (10a) verhindert ein seitliches Einknicken der Ausschüttfeder (9) während und am Ende des Ausschütthubs des Vortriebsglieds (6).

Der Autoinjektor weist eine Sperrhülse (7) auf. Im Auslieferungszustand oder in der Ausgangsposition des Autoinjektors wird die Eingriffsnocke (5a) von einem Innenumfang der Sperrhülse (7), die an der Führungsnocke (5b) anliegt, in einem Eingriff oder in einer Kopplung mit der Ausnehmung (6a) der Vortriebglieds (6) gehalten.

Die Sperrhülse (7) ist mit der Nadelschutzhülse (3), insbesondere mit einem proximalen Ende der Nadelschutzhülse (3) drehbar verbunden, oder liegt an der Nadelschutzhülse (3), insbesondere an dem proximalen Ende der Nadelschutzhülse (3) an. Eine Ausschüttfeder (9) stützt sich mit ihrem distalen Ende an der Sperrhülse (7) und mit ihrem proximalen Ende an der Verschlusskappe (10) ab.

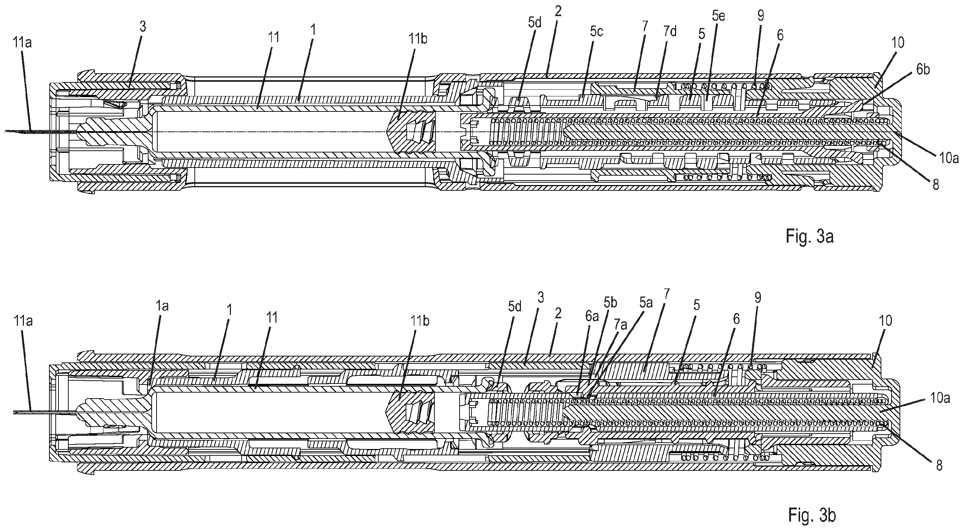

Die Sperrhülse (7) und der Mechanikhalter (5) weisen eine lösbare Rotationssicherung auf. Die Sperrhülse (7) umfasst eine Rippe (5f), welche mit einer an dem Mechanikhalter (5) vorgesehene erste Nut (7b) in und aus einem Eingriff oder in oder aus einer Kopplung gelangen kann. In der Ausgangsposition der Nadelschutzhülse (3) ist die Rippe (5f) des Mechanikhalters (5) in dem Eingriff oder in der Kopplung mit der ersten Nut (7b) der Sperrhülse (7). Durch das Verschieben der Nadelschutzhülse in die betätigte Position der Nadelschutzhülse (3) (Figuren 3a und 3b) gelangt die Rippe (5f) aus dem Eingriff oder aus der Kopplung mit der ersten Nut (7b) der Sperrhülse (7). Die Rotationssicherung zwischen der Sperrhülse (7) und dem Mechanikhalter (5) ist zumindest in der betätigen Position der Nadelschutzhülse (3) gelöst.

Die Sperrhülse (7) weist eine Führungskulisse (7e) auf, welche mit der Führungsnocke (5b) des Mechanikhalters (5) in oder aus einem Eingriff oder in oder aus einer Kopplung sein kann. Durch das Verschieben der Nadelschutzhülse in die betätigte Position der Nadelschutzhülse (3) (Figuren 3a und 3b) gelangt die Führungsnocke (5b) des Mechanikhalters (5) in den Eingriff oder in die Kopplung mit der Führungskulisse (7e) der Sperrhülse (7).

Die Sperrhülse (7) weist ferner ein federnd oder elastisch angeordnetes Verriegelungsglied (7d) auf, welches mit einem an dem Mechanikhalter (5) vorgesehenen Verriegelungsanschlag (5c) eine Verriegelungseinrichtung bildet. Das Verriegelungseinrichtung dient der Verriegelung der Nadelschutzhülse (3) in ihrer Nadelschutzposition, wie in den Figuren 6a und 6b dargestellt ist.

Zur Verabreichung des Produkts aus der Spritze (11) wird die Abziehkappe (4) von dem Autoinjektor zusammen mit der Nadelschutzkappe (12) entfernt. Das distale Ende der Nadelschutzhülse (3) wird an die Einstichstelle eines Patienten angesetzt, wobei das Gehäuse (2) zu der Einstichstelle hin verschoben wird, wodurch sich die Nadelschutzhülse (3) aus ihrer Ausgangsposition um den Betätigungshub in die proximale Richtung relativ zu dem Gehäuse (2) in die betätigte Position bewegt. Hierdurch wird die Nadelschutzfeder (9) gespannt, wobei die Sperrhülse (7) von der Nadelschutzhülse (3) um den Betätigungshub mitgenommen wird. Die Sperrhülse (7) weist eine Ausnehmung (7a) auf, die durch Verschieben der Sperrhülse (7) um den Betätigungshub entlang der Längsachse L auf die Position der Führungsnocke (5b) gebracht wird. Hierdurch wird die Eingriffsnocke (5a) aus dem Eingriff mit dem Vortriebsglied (6), insbesondere aus dem Eingriff mit der Ausnehmung (6a) des Vortriebsglieds (6) mit einer Bewegung quer zu und von der Längsachse L weg bewegt, wobei gleichzeitig die Führungsnocke (5b) in den Eingriff mit der Führungskulisse (7e) der Sperrhülse (7) gelangt. Hierdurch wird das Vortriebsglied (6) für die Bewegung um den Ausschütthub in die Ausschüttrichtung freigegeben (Figuren 3a und 3b). Zudem gelangt oder ist die die erste Nut (7b) der Sperrhülse (7) aus dem Eingriff mit der Rippe (5f) des Mechanikhalters (5), wobei die Rotationssicherung zwischen der Sperrhülse (7) und dem Mechanikhalter (5) gelöst ist.

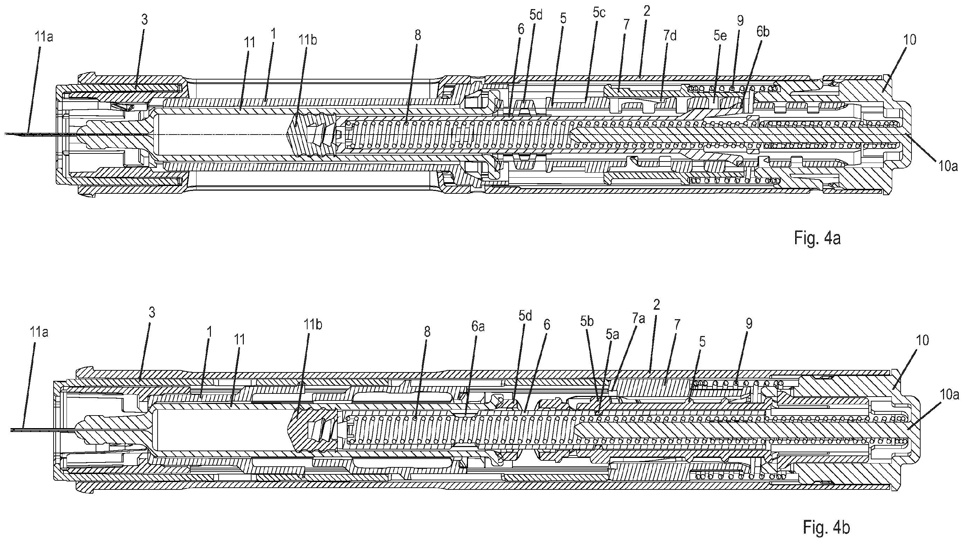

Das Vortriebsglied (6) bewegt sich aufgrund der Entspannung der Federkraft der Ausschüttfeder (8) in die distale Richtung, um das Produkt aus der Spritze (11) auszuschütten. Während der Produktausschüttung wird ein Ausschüttsignal, insbesondere ein akustisches Ausschüttsignal erzeugt, wie in den Figuren 4a, 4b, 7a, 7b, 8a und 8b gezeigt ist.

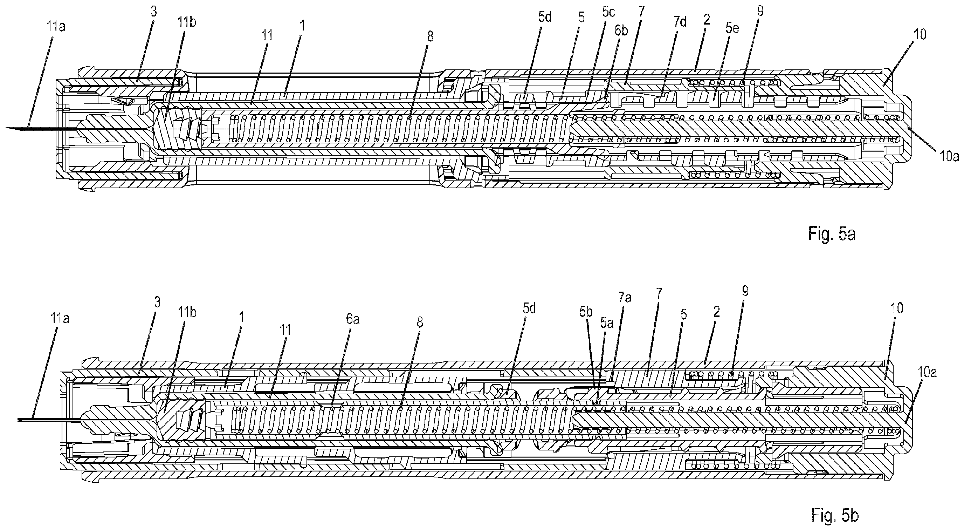

In bevorzugten Ausführungsformen gemäss den Figuren 4a und 4b weist das Vortriebsglied (6) ein Signalglied (6b) auf. In diesem Beispiel umfasst das Vortriebsglied (6) einen federnden oder elastischen Arm, welcher das Signalglied (6b) umfasst. Das Signalglied (6b) ist ein radial nach aussen ragender Vorsprung, welcher mit entlang der Längsachse vorgesehenen Öffnungen (5e) in dem Mechanikhalter (5) derart zusammen wirkt, dass während dem ganzen oder zumindest teilweisen Ausschüttvorgang ein Ausschüttsignal, insbesondere ein akustisches Signal erzeugt wird. In diesem Beispiel sind die Öffnungen (5e) in dem Mechanikhalter (5) gleichmässig voneinander beabstandet. Somit kann sichergestellt werden, dass während dem ganzen oder zumindest teilweisen Ausschütthub des Kolbens oder des Vortriebsglieds dem Patienten angezeigt wird, dass der Ausschüttvorgang im Gange ist oder von statten geht. Das Ende der Produktausschüttung (Figuren 5a und 5b) wird derart angezeigt, das kein Ausschüttsignal mehr erzeugt wird oder das Ausschüttsignal verstummt ist, da keine axiale Relativbewegung zwischen dem Signalglied (6b) und den Öffnungen (5e) stattfindet.

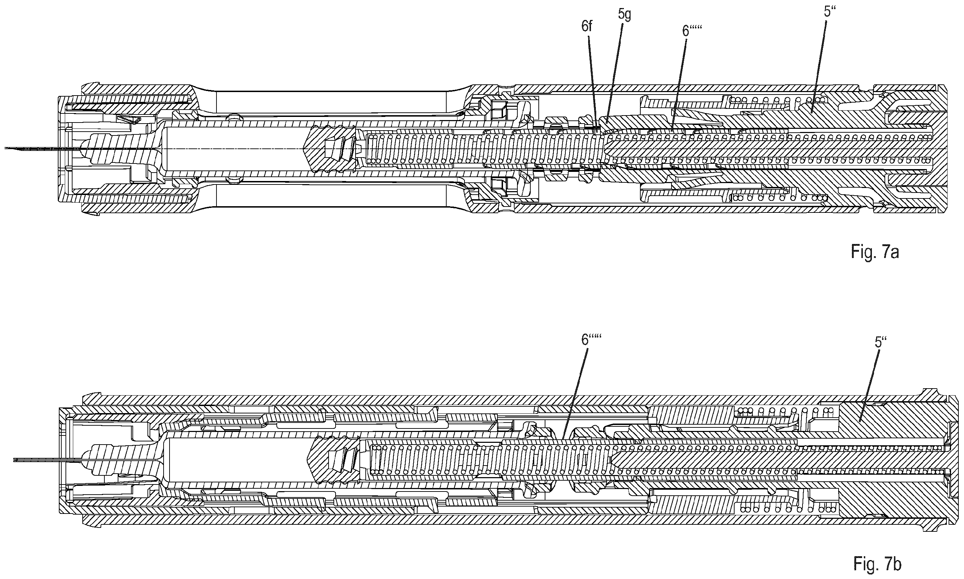

In alternativen Ausführungsformen gemäss den Figuren 7a und 7b weist das Vortriebsglied (6‴ʺ) mehrere Öffnungen (6f) auf. In diesem Beispiel sind die Öffnungen (6f) gleichmässig voneinander beabstandet. Der Mechanikhalter (5") weist das Signalglied (5g) auf, wobei das Signalglied (5g) in diesem Beispiel an einem federnden oder elastischen Arm des Mechanikhalters (5") vorgesehen ist. Das Signalglied (5g) ist ein radial nach aussen ragender Vorsprung, welcher mit entlang der Längsachse vorgesehenen Öffnungen (6f) in dem Vortriebsglied (6‴ʺ) derart zusammen wirkt, dass während dem ganzen oder zumindest teilweisen Ausschüttvorgang ein Ausschüttsignal, insbesondere ein akustisches Signal erzeugt wird.

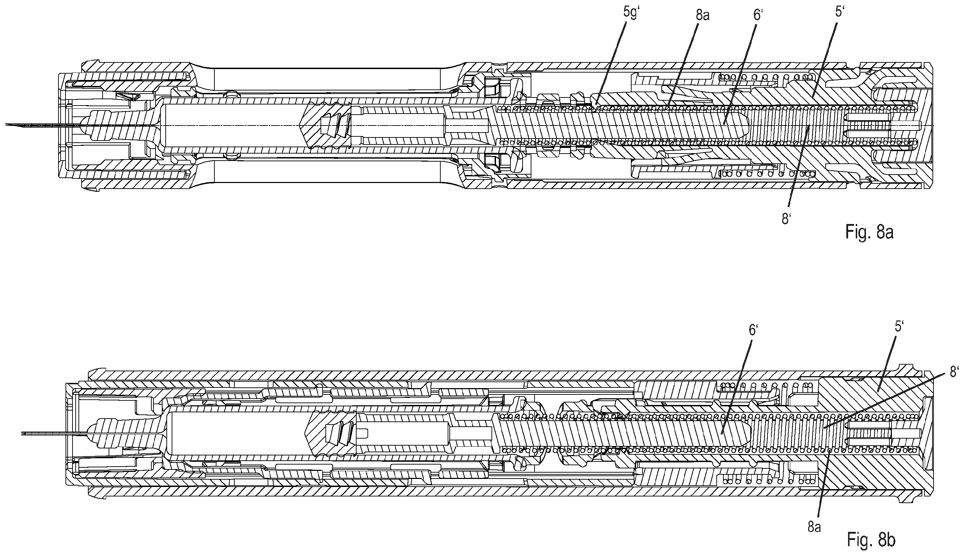

In alternativen Ausführungsformen gemäss den Figuren 8a und 8b weist der Mechanikhalter (5') ein Signalglied (5g') auf. In diesem Beispiel ist das Signalglied (5g') an einem federnden oder elatischen Arm des Mechanikhalters (5') angeordnet. Das Signalglied (5g') ist als ein radial nach innen ragender Vorsprung ausgebildet sein, welcher über die Ausschüttfeder (8'), insbesondere über die Windungen (8a) der Ausschüttfeder (8') gleiten kann, um ein akustisches Signal zu erzeugen. Alternativ kann die Aussschüttfeder innerhalb des Vortriebsglied angeordnet sein, wobei das Vortriebsglied eine entlang der Längsachse angeordnete Öffnung, insbesondere eine Längsöffnung aufweist, wobei das Signalglied durch die Öffnung, insbesondere durch die Längsöffnung des Vortiebsglied ragen kann, um mit der Ausschüttfeder, insbesondere den Windungen der Ausschüttfeder derart zusammen zu wirken, dass ein Ausschüttsignal erzeugt wird.

Durch Abnehmen des Autoinjektors von der Injektionsstelle kann die Nadelschutzfeder (9), die Sperrhülse (7) und die Nadelschutzhülse (3) aus der betätigten Position in die Nadelschutzposition (Figuren 6a und 6b) um den Nadelschutzhub bewegen, wobei die Sperrhülse (7) relativ zu dem Mechanikhalter (5) dreht. Durch diese Relativdrehung zwischen der Führungskulisse (7e) und der Führungsnocke (5b) gelangt das Verriegelungsglied (7d) der Sperrhülse (7) in die axiale Flucht mit dem Verriegelungsanschlag (5c) des Mechanikhalters (5). Zudem gelangt eine zweite Nut (7c) der Sperrhülse in die axiale Flucht mit der Rippe (5f) des Mechanikhalters (5), um die Nadelschutzhülse (3) in die Nadelschutzposition (Figuren 6a und 6b) zu führen. In der Nadelschutzposition (Figuren 6a und 6b) verhindert die Verriegelungseinrichtung ein Zurückschieben der Nadelschutzhülse (3) in ihre betätigte Position. Bei dem Versuch, die Nadelschutzhülse (3) aus der Nadelschutzposition in die betätigte Position zurückzuschieben, stösst das Verriegelungsglied (7d) der Sperrhülse (7) an den Verriegelungsanschlag (5c) des Mechanikhalters (5), wobei die Bewegung der Nadelschutzhülse 3 in die betätigte Position verhindert wird.

### Bezugszeichenliste

- 1: Produktbehälterhalter, Spritzenhalter
- 1a: Eingriffsglied, Schulter
- 2: Gehäuse
- 3: Nadelschutzhülse
- 4: Abziehkappe
- 4a: Schnapphaken
- 5; 5'; 5": Mechanikhalter
- 5a: Eingriffsnocke
- 5b: Führungsnocke
- 5c: Verriegelungsanschlag
- 5d: Haltefeder
- 5e: Öffnungen
- 5f: Rippe
- 5g; 5g': Signalglied
- 6; 6'; 6"; 6‴; 6ʺʺ; 6‴ʺ: Vortriebsglied
- 6a: Ausnehmung
- 6b: Signalglied
- 6c: Gegenzahnung
- 6d: Gegenquetschelement
- 6e: Gegengewinde
- 6f: Öffnungen
- 7: Sperrhülse
- 7a: Aussparung
- 7b: erste Nut
- 7c: zweite Nut
- 7d: Verriegelungsglied
- 7e: Führungskulisse
- 8;8': Ausschüttfeder
- 8a: Windungen
- 9: Nadelschutzfeder
- 10: Verschlusskappe
- 10a: Führung
- 11: Produktbehälter, Spritze
- 11a: Nadel
- 11b: Kolben
- 12: Nadelschutzkappe
- 13; 13'; 13": Adapter
- 13a: Zahnung
- 13b: Quetschelement
- 13c: Gewinde
- L: Längsachse

## Patentansprüche

1. Autoinjektor zur Ausschüttung eines flüssigen Produkts, insbesondere Medikaments, umfassend:
a) ein Gehäuse (2) oder ein mit dem Gehäuse (2) axialfest und drehfest verbundenen Mechanikhalter (5; 5'; 5") und einen in dem Gehäuse (2) oder in dem Mechanikhalter (5; 5'; 5") angeordneten Produktbehälter (11), insbesondere Spritze, der einen verschiebbaren Kolben (11b) aufweist, wobei der Kolben (11b) zur Ausschüttung des in dem Produktbehälter (11) enthaltenen Produkts in eine Ausschüttrichtung verschiebbar ist,
b) ein Vortriebsglied (6; 6'; 6"; 6‴; 6ʺʺ; 6‴ʺ), das während der Produktausschüttung auf den Kolben (11b) wirkt, und eine Ausschüttfeder (8), die auf das Vortriebsglied (6; 6'; 6"; 6‴; 6ʺʺ; 6‴ʺ) wirkt,
c) eine Nadelschutzhülse (3) und eine Nadelschutzfeder (9), welche für die Auslösung der Produktausschüttung aus ihrer Ausgangsposition relativ zu dem Gehäuse (2) oder relativ zu dem Mechanikhalter (5; 5'; 5") und entlang der Längsachse (L) des Autoinjektors in eine proximale Richtung um einen Betätigungshub verschiebbar ist, wobei die Nadelschutzfeder (9) gespannt und die Produktausschüttung ausgelöst wird,
d) eine Sperrhülse (7), welche geometrisch oder kinematisch zwischen der Nadelschutzfeder (9) und der Nadelschutzhülse (3) angeordnet ist, wobei die Sperrhülse (7) von der Nadelschutzhülse (3) in die proximale Richtung mitgenommen wird, wenn die Nadelschutzhülse (3) aus ihrer Ausgangsposition in die proximale Richtung verschoben wird, wobei
e) die Nadelschutzhülse (3) nach der Produktausschüttung, wenn das Vortriebsglied (6; 6'; 6"; 6‴; 6ʺʺ; 6‴ʺ) oder der Kolben (11b) um den Ausschütthub in die distale Richtung verschoben ist, von der Nadelschutzhülsenfeder (9) relativ zu dem Gehäuse (2) oder relativ zu dem Mechanikhalter (5; 5'; 5") in eine Nadelschutzposition verschiebbar ist, in der die Nadelschutzhülse (3) distal über die Nadelspitze einer Nadel (11a) des Produktbehälters (11) steht,
**dadurch gekennzeichnet, dass** die Sperrhülse (7) und das Gehäuse (2) oder der Mechanikhalter (5; 5'; 5") in einen Eingriff oder in eine Kopplung gelangen können, wobei der Eingriff oder die Kopplung dazu ausgelegt, dass die Sperrhülse (7) relativ zu dem Gehäuse (2) oder relativ zu dem Mechanikhalter (5; 5'; 5") dreht, wenn die Nadelschutzhülse (3) relativ zu dem Gehäuse (2) oder relativ zu dem Mechanikhalter (5; 5'; 5") in die Nadelschutzposition verschoben wird, wobei eine Verriegelungseinrichtung an der Sperrhülse (7) und an dem Gehäuse (2) oder dem Mechanikhalter (5; 5'; 5") vorgesehen ist, welche in der Nadelschutzposition die Nadelschutzhülse (3) relativ zu dem Gehäuse (2) oder relativ zu dem Mechanikhalter (5; 5'; 5") gegen ein Zurückschieben in die proximale Richtung so verriegelt, dass die die Nadelspitze nicht aus dem distalen Ende der Nadelschutzhülse (3) hervortreten kann.

2. Autoinjektor nach Anspruch 1, **dadurch gekennzeichnet, dass** die Sperrhülse (7) ein federndes oder elastisches Verriegelungsglied (7d) und das Gehäuse (2) oder der Mechanikhalter (5; 5'; 5") ein Verriegelungsanschlag (5c) oder alternativ, dass das Gehäuse (2) oder der Mechanikhalter (5; 5'; 5") ein federndes oder elastisches Verriegelungsglied und die Sperrhülse (7) ein Verriegelungsanschlag aufweisen, um die Nadelschutzhülse (3) in der Nadelschutzposition zu verriegeln.

3. Autoinjektor nach Anspruch 1 oder 2, **dadurch gekennzeichnet, dass** das Gehäuse (2) oder der Mechanikhalter (5; 5'; 5") eine Eingriffsnocke (5a) aufweist, welche lösbar in das Vortriebsglied (6; 6'; 6"; 6‴; 6ʺʺ; 6‴ʺ) eingreift, wodurch das Vortriebsglied axialfest mit dem Gehäuse (2) oder dem Mechanikhalter (5; 5'; 5") gekoppelt ist, wobei die axialfeste Kopplung oder der axialfeste Eingriff zwischen dem Vortriebsglied (6; 6'; 6"; 6‴; 6ʺʺ; 6‴ʺ) und dem Gehäuse (2) oder dem Mechanikhalter (5; 5'; 5") gelöst ist, wenn die Eingriffsnocke (5a) aus dem Eingriff oder aus der Kopplung mit dem Vortriebsglied (6; 6'; 6"; 6‴; 6ʺʺ; 6‴ʺ) ist, um das Produkt aus dem Produktbehälter (11) auszuschütten.

4. Autoinjektor nach einem der vorhergehenden Ansprüche, **dadurch gekennzeichnet, dass** das Gehäuse (2) oder der Mechanikhalter (5; 5'; 5") eine Führungsnocke (5b) aufweist, welche mit einer Führungskulisse (7b) an der Sperrhülse (7) in den Eingriff oder in die Kopplung gelangen kann, um die Sperrhülse (7) relativ zu dem Gehäuse (2) oder relativ zu dem Mechanikhalter (5; 5'; 5") zu drehen oder alternativ, dass das Gehäuse (2) oder der Mechanikhalter (5; 5'; 5") eine Führungskulisse aufweist, welche mit einer Führungsnocke an der Sperrhülse (7) in den Eingriff oder in die Kopplung gelangen kann, um die Sperrhülse (7) relativ zu dem Gehäuse (2) oder relativ zu dem Mechanikhalter (5; 5'; 5") zu drehen.

5. Autoinjektor nach den Ansprüchen 3 und 4, **dadurch gekennzeichnet, dass** die Eingriffsnocke (5a) und die Führungsnocke (5b) so aufeinander abgestimmt sind, dass die Kulissensteuerung, insbesondere die Rotationsführung zwischen der Sperrhülse (7) und dem Gehäuse (2) oder dem Mechanikhalter (5) bereits im Eingriff oder gekoppelt ist, wenn die Eingriffsnocke (5a) noch nicht vollständig aus dem Eingriff oder aus der Kopplung mit dem Vortriebsglied (6; 6'; 6"; 6‴; 6ʺʺ; 6‴ʺ) gelöst ist.

6. Autoinjektor nach einem der vorhergehenden Ansprüche, **dadurch gekennzeichnet, dass** in der Ausgangsposition der Nadelschutzhülse (3) eine lösbare Rotationsicherung zwischen der Sperrhülse (7) und dem Gehäuse (2) oder dem Mechanikhalter (5; 5'; 5") vorgesehen ist, um eine Rotation der Sperrhülse (7) relativ zu dem Gehäuse (2) oder dem Mechanikhalter (5; 5'; 5") zu verhindern.

7. Autoinjektor nach Anspruch 6, **dadurch gekennzeichnet, dass** die Rotationssicherung zwischen der Sperrhülse (7) und dem Gehäuse (2) oder dem Mechanikhalter (5; 5'; 5") gelöst ist, wenn die Nadelschutzhülse (2) in ihrer betätigten Position ist, wobei in der betätigten Position das Vortriebsglied (6; 6'; 6"; 6‴; 6ʺʺ; 6‴ʺ) zur Produktausschüttung in die proximale Richtung bewegbar ist und die Sperrhülse (7) relativ zu dem Gehäuse (2) und dem Mechanikhalter (5; 5'; 5") drehbar ist, um die Nadelschutzhülse (3) in der Nadelschutzposition zu verriegeln.

8. Autoinjektor nach einem der vorhergehenden Ansprüche, **dadurch gekennzeichnet, dass** die Nadelschutzhülse (3) relativ zu dem Gehäuse (2) oder zu dem Mechanikhalter (5; 5'; 5") drehfest und axial bewegbar angeordnet ist.

9. Autoinjektor nach einem der vorhergehenden Ansprüche, **dadurch gekennzeichnet, dass** das Vortriebsglied (6) ein federndes oder elastisches Signalglied (6b) aufweist, welches mit an dem Gehäuse (2) oder an dem Mechanikhalter (5) entlang der Längsachse (L) angeordneten Öffnungen (5e) derart zusammenwirkt, dass während dem ganzen oder zumindest teilweisen Ausschütthub des Vortriebglieds (6) oder des Kolbens (11b) ein Ausschüttsignal erzeugt wird.

10. Autoinjektor nach Anspruch 9, **dadurch gekennzeichnet, dass** die Öffnungen (5e) gleichmässig oder ungleichmässig entlang der Längsachse (L) des Gehäuses (2) oder des Mechanikhalters (5) angeordnet sind.

11. Autoinjektor nach Anspruch 9 oder 10, **dadurch gekennzeichnet, dass** das Signalglied (6b) an einem proximalen Ende oder an einem proximalen Bereich des Vortriebsglieds (6) vorgesehen ist.

12. Autoinjektor nach einem der Ansprüche 9 bis 11, **dadurch gekennzeichnet, dass** das Signalglied (6b) als ein radial nach aussen ragender Vorsprung ausgebildet ist.

13. Autoinjektor nach einem der Ansprüche 9 bis 12, **dadurch gekennzeichnet, dass** der Autoinjektor eine Verstärkungseinrichtung aufweist, um das Ausschüttsignal zu verstärken.

14. Autoinjektor nach einem der vorhergehenden Ansprüche, **dadurch gekennzeichnet, dass** zwischen dem Vortriebsglied (6; 6'; 6"; 6‴; 6ʺʺ; 6‴ʺ) und dem Kolben (11b) des Produktbehälters (11) ein Adapter (13, 13', 13") angeordnet ist, um in dem Ausgangszustand den Abstand zwischen dem Vortriebsglied (6; 6'; 6"; 6‴; 6ʺʺ; 6‴ʺ) und dem Kolben (11b) einzustellen oder/und beim Start der Produktausschüttung die kinetische Energie des Vortriebsglied (; 6'; 6"; 6‴; 6ʺʺ; 6‴ʺ) zumindest teilweise zu absorbieren.

15. Autoinjektor nach Anspruch 14, **dadurch gekennzeichnet, dass** der Adapter (13', 13") axial bewegbar innerhalb des Vortriebsglieds (6; 6'; 6"; 6‴; 6ʺʺ; 6‴ʺ) angeordnet ist.
